Europäisches Patentamt

European Patent Office

Office européen des brevets

(11) Veröffentlichungsnummer: **0 525 333 A1**

# EUROPÄISCHE PATENTANMELDUNG

(21) Anmeldenummer: **92109329.0**

(22) Anmeldetag: **03.06.92**

(51) Int. Cl.5: **A61K 9/16**, A61K 9/50,
A61K 47/36

(30) Priorität: **25.06.91 DE 4120918**

(43) Veröffentlichungstag der Anmeldung:
**03.02.93 Patentblatt 93/05**

(84) Benannte Vertragsstaaten:
**CH DE DK FR GB IT LI**

(71) Anmelder: **BASF Aktiengesellschaft
Carl-Bosch-Strasse 38
W-6700 Ludwigshafen(DE)**

(72) Erfinder: **Schneider, Joachim U., Dr.
Plauserstrasse 17
W-6719 Wiesenheim(DE)**
Erfinder: **Schwarz, Gerhard, Dr.
Hainbachweg 8
W-6721 Harthausen(DE)**
Erfinder: **Grafen, Paul, Dr.
Suedtiroler Ring 20
W-6719 Weisenheim(DE)**
Erfinder: **Bewert, Wolfgang, Dr.
Lorscher Ring 8c
W-6710 Frankenthal(DE)**
Erfinder: **Schumacher, Horst, Dr.
Battenberger Weg 4
W-6719 Bobenheim(DE)**

(54) **Stärken mit hohem Amylosegehalt zum Herstellen von pulverförmigen Präparaten.**

(57) Pulverförmige Präparate aus einer wasserunlöslichen Kernsubstanz und einer Schutzhülle, erhältlich durch
- Dispergieren der Kernsubstanz in der wäßrigen Lösung von Amylose oder einer Stärke mit einem Amylosegehalt von über 40 Gew.-% als filmbildenden Polymeren für die Schutzhülle,
- Versprühen dieser Dispersion unter Mitverwendung von hydrophobierter Kieselsäure als Sprühhilfsmittel und
- anschließende Trocknung der versprühten Teilchen.

EP 0 525 333 A1

EP 0 525 333 A1

Die vorliegende Erfindung betrifft pulverförmige Präparate aus einer wasserunlöslichen Kernsubstanz und einer Schutzhülle, erhältlich durch

- Dispergieren der Kernsubstanz in der wäßrigen Losung von Amylose oder einer Stärke mit einem Amylosegehalt von über 40 Gew.-% als filmbildenden Polymeren für die Schutzhülle,
- Versprühen dieser Dispersion unter Mitverwendung von hydrophobierter Kieselsäure als Sprühhilfsmittel und
- anschließende Trocknung der versprühten Teilchen.

Außerdem betrifft die Erfindung ein Verfahren zur Herstellung der pulverförmigen Präparate aus einer wasserunlöslichen Kernsubstanz und einer Schutzhülle.

Pulverförmige Präparate aus einer wasserunlöslichen Kernsubstanz wie insbesondere Vitaminen und Carotinoiden und einer Schutzhulle sind allgemein bekannt und werden in der weiterverarbeitenden pharmazeutischen Industrie sowie in der Nahrungs- und Futtermittelindustrie verwendet.

Aus der US-A 3 499 962 sind pulverförmige Präparate aus wasserunlöslichen Kernsubstanzen wie z.B. Parfümen oder Vitaminen und einer Schutzhülle aus Stärke mit einem Amylosegehalt über 40 Gew.-% bekannt und in der GB-A 10 72 795 ist die Verwendung von Amylose als filmbildendes Polymeres zur Einkapselung von Vitaminen beschrieben. Nachteilig ist jedoch die Überführung der Dispersion in die pulverförmigen Präparate, die entweder verfahrenstechnisch zu aufwendig ist oder zu kleine Partikelgrößen liefert.

Aus der EP-A 00 74 050 ist die Herstellung von Trockenpulvern durch Versprühen einer Dispersion unter Zugabe von hydrophober Kieselsäure als Sprühhilfsmittel und anschließender Trocknung bekannt.

Der Erfindung lagen pulverförmige Präparate der genannten Art zugrunde, die verfahrenstechnisch einfacher zugänglich sind und anwendungstechnisch bessere Eigenschaften haben als die bisher bekannten Zubereitungen.

Demgemäß wurden die eingangs definierten pulverförmigen Präparate aus einer wasserunlöslichen Kernsubstanz und einer Schutzhülle sowie ein Verfahren zur Herstellung dieser pulverförmigen Präparate gefunden.

Erfindungsgemäß einzusetzende filmbildende Polymere sind Stärke mit einem Amylosegehalt über 40 Gew.-% oder bevorzugt Amylose, die im allgemeinen in Mengen von 30 bis 99 Gew.-%, bezogen auf das pulverförmige Präparat, eingesetzt werden.

Die Amylose kann aus Maisstärke gewonnen werden, die einen Anteil von 20 bis 30 Gew.-% Amylose neben 80 bis 70 Gew.-% Amylopektin enthält. Stärke mit einem Amylosegehalt von mindestens 40 Gew.-% kann aus neuartigen Züchtungen von hochamylosehaltigen Maissorten oder aus Markerbsen gewonnen werden.

Die erfindungsgemäß zu verwendende Amylose oder hochamylosehaltige Stärke haben den Vorteil, daß sie erst bei hoher Temperatur wasserlöslich sind und beim Erkalten als wasserunlöslicher Film ausfallen. Derartige Schutzhüllen zeichnen sich daher durch eine hohe thermische und mechanische Stabilität sowie durch Wasserfestigkeit aus. Die auf diese Weise hergestellten Präparate lassen sich somit auch unter starker Beanspruchung wie beim Extrudieren oder Pressen weiterverarbeiten.

Eine Lösung der filmbildenden Polymeren kann sowohl diskontinuierlich bei Temperaturen von 150 bis 160°C im Autoklaven als auch bevorzugt kontinuierlich mit Dampf von 5 bis 10 bar mittels einer Kochdüse (Jet-Kocher) erzeugt werden.

Als wasserunlösliche Kernsubstanzen kommen bevorzugt pharmazeutische Wirkstoffe, Aromastoffe und vor allem Vitamine und Carotinoide in Betracht. Weiterhin eignen sich beispielsweise Fette, Öle und Parfüme.

Die Menge der einzukapselnden wasserunlöslichen Kernsubstanz beträgt in der Regel 1 bis 40 Gew.-%, bezogen auf das pulverförmige Präparat.

Zur Herstellung der Dispersion wird die Kernsubstanz in der wäßrigen Lösung des filmbildenden Polymeren dispergiert, wobei die Dispersion Additive wie Antioxidantien, Emulgatoren und/oder Konservierungsstoffe, Weichmacher, Stabilisatoren, Komplexbildner oder weitere Filmbildner enthalten kann.

Die anschließende Versprühung der Dispersion erfolgt unter Mitverwendung eines Sprühhilfsmittels, wobei gemäß der Erfindung hydrophobierte Kieselsäure verwendet wird (Die Mühle und Mischfuttertechnik, 114, 3 (1977)). Bei der hydrophobierten Kieselsäure handelt es sich um Kieselsäurepartikel, deren freie Hydroxylgruppen an der Oberfläche mit einer hydrophoben Verbindung wie insbesondere einem Halogenalkylsilan, beispielsweise Dimethyldichlorsilan, umgesetzt wurden.

Die Zufuhr des Sprühhilfsmittels und dessen Zerstäubung erfolgt zusammen mit Luft mit etwa 2 bis 5 $m^3$/kg Sprühhilfsmittel, wobei die notwendigen Mengen des Sprühhilfsmittels nur das 0,02- bis 0,15-fache der Dispersion betragen.

Bevorzugt verwendet man einen Sprühturm und führt die hydrophobierte Kieselsäure unter gleichmäßi-

2

ger Verteilung in den Sprühraum bei Temperaturen von 25 bis 30°C ein.

Zweckmäßigerweise führt man das Sprühhilfsmittel oberhalb der Zerstäubungsvorrichtung zu, beispielsweise können Düsen oder schnellrotierende Zerstäuberscheiben benutzt werden.

Die Temperatur der zu zerstäubenden Dispersion ist keine kritische Größe. Sie liegt üblicherweise bei 60 bis 90°C.

Durch das direkte Einbringen der hydrophobierten Kieselsäure in die Sprühzone wird eine mechanische Beanspruchung der Partikel weitgehend vermieden. Der während des Sprühens erzeugte dünne hydrophobe Film des Sprühhilfsmittels stabilisiert die Partikel soweit, daß eine Agglomeration der Partikel bei Berührung im nicht erstarrten Zustand verhindert wird, so daß die direkte Trocknung auf einem sich anschließenden Wirbelbett-Trockner in an sich bekannter Weise möglich ist.

Die Trocknung erfolgt im allgemeinen bei 30 bis 80°C, da bei dieser Temperatur der Trocknungsluft der Überschuß der hydrophobierten Kieselsäure entweicht.

Die nach dem erfindungsgemäßen Verfahren auf einfache Weise erhältlichen pulverförmigen Präparate zeichnen sich durch hohe Stabilitäten wie z.B. Heißwasserunlöslichkeit aus und bestehen aus Teilchen mit gut ausgebildeter Oberfläche. Beim Versprühen richtet man es in an sich bekannter Weise so ein, daß die Teilchen eine mittlere Korngröße von 100 bis 600 $\mu$m, insbesondere von 180 bis 350 $\mu$m haben. Pulver mit diesem Korngrößenbereich der Teilchen gewährleisten einen ausreichenden Schutz der eingekapselten Kernsubstanz und eignen sich besonders gut für die Weiterverarbeitung, beispielsweise zu Nahrungs- oder Futtermitteln.

Beispiele

Herstellung pulverförmiger Präparate

Eine Suspension von a Gew.-% eines filmbildenden Polymeren P in b Gew.-% Wasser wurde für die Dauer von t Minuten auf $T_1$°C erhitzt und nach weiteren 20 Minuten innerhalb von 90 Minuten auf 80°C abgekühlt. Anschließend wurde das Gemisch mit einer Lösung aus c Gew.-% Additiven A in d Gew.-% Wasser versetzt und für die Dauer von 10 Minuten bei 80°C gehalten.

Danach wurden e Gew.-% Kernsubstanz K zugegeben und innerhalb von 10 Minuten in die Lösung eingerührt.

Anschließend wurde die Dispersion bei einem Druck von f bar und einer Temperatur von 80°C in mit hydrophobierter Kieselsäure beladener Luft versprüht. Dabei betrug die Zugabe an Kieselsäure 10 kg/h und die Sprühleistung 80 kg/h wäßriger Dispersion. Die übliche Aufarbeitung durch Trocknung im Wirbelbett bei 30°C (Beispiele 1 bis 4) bzw. 60°C (Beispiel 5) lieferte das Pulver mit einem Restwassergehalt von g % und einem Wirkstoffgehalt von h IE/g bzw. i %.

Die Bewertung der Partikel erfolgte durch eine Ermittlung der Korngrößenverteilung durch die Siebanalyse nach ASTM.

In allen Fällen wurden Teilchen mit Korngrößen von 100 bis 600 $\mu$m und mit einem Maximum der Teilchengrößeverteilung bei etwa 180 bis 350 $\mu$m erhalten.

Die Einzelheiten der Versuche sind Tabelle 1 und 2 zu entnehmen.

Bedeutung der Abkürzungen in der Tabelle

Polymer P

P/1     Maisstärke mit einem Amylosegehalt von 70 %
P/2     Markerbsenstärke mit einem Amylosegehalt von 75 %

Additive A

A/1     vorgequollene Gelatine (Filmbildner)
A/2     Glucosesirup (Weichmacher)

Kernsubstanz K

K/1     Vitamin-A-Acetat (2,23 Mio IE/g), stabilisiert mit Ethoxyquin
K/2     Vitamin-A-Acetat (2,12 Mio IE/g), stabilisiert mit Ethoxyquin
K/3     Vitamin-E-Acetat (24,7 %)

K/4    Citranaxanthin (33,2 %)
K/5    Vitamin-A-Acetat (2,15 Mio IE/g), stabilisiert mit Ethoxyquin

Tabelle 1
Herstellung pulverförmiger Präparate

|  | Beispiele | | | | |
|---|---|---|---|---|---|
|  | 1 | 2 | 3 | 4 | 5* |
| Polymer P | P/1 | P/2 | P/1 | P/1 | P/1 |
| a [Gew.-%] | 153,3 | 102 | 221,6 | 233 | 15,3 |
| b [Gew.-%] | 550 | 550 | 900 | 1000 | 58 |
| t [min] | 55 | 55 | 20 | 20 |  |
| $T_1$ [°C] | 150 | 150 | 150 | 150 | 163 |
| Additive A | A/1 u. A/2 | A/2 |  |  |  |
| c [Gew.-%] | 17,2 u. 67,4 | 56,3 |  |  |  |
| d [Gew.-%] | 200 | 200 |  |  |  |
| Kernsubstanz K | K/1 | K/2 | K/3 | K/4 | K/5 |
| e [Gew.-%] | 77,7 | 51,8 | 85 | 54,6 | 5,1 |
| f [bar] | 4,8 | 4,8 | 5 | 5 | 12 |
| g [%] | 2 | 3,2 | 2,7 | 2,3 | 4,7 |
| h [IE/g] | 603000 | 506000 |  |  | 532200 |
| i [%] |  |  |  | 24,7 | 7,2 |

* kontinuierlicher Stärkeaufschluß im Jet-Kocher bei 6 bar

Tabelle 2

| Korngrößenverteilung der pulverförmigen Präparate | | | | | |
|---|---|---|---|---|---|
|  | Beispiele | | | | |
| Korngrößenverteilung [μm] | 1 | 2 | 3 | 4 | 5 |
| > 500 | 2,6 | 2,6 | 10,1 | 6,4 | 4,2 |
| > 425 | 3,8 | 2,3 | 11,5 | 6,2 | 11,2 |
| > 355 | 10,1 | 4,1 | 14,1 | 9,0 | 21,8 |
| > 250 | 55,2 | 19,0 | 28,4 | 30,3 | 38,0 |
| > 180 | 16,5 | 37,7 | 25,6 | 31,6 | 18,8 |
| > 125 | 7,5 | 24,7 | 9,3 | 15,5 | 5,4 |
| > 106 | 2,1 | 7,5 | 1,0 | 0,7 | 0,6 |

**Patentansprüche**

1. Pulverförmige Präparate aus einer wasserunlöslichen Kernsubstanz und einer Schutzhülle, erhältlich durch
   - Dispergieren der Kernsubstanz in der wäßrigen Lösung von Amylose oder einer Stärke mit einem Amylosegehalt von über 40 Gew.-% als filmbildenden Polymeren für die Schutzhülle,
- Versprühen dieser Dispersion unter Mitverwendung von hydrophobierter Kieselsäure als Sprühhilfsmittel und
- anschließende Trocknung der versprühten Teilchen.

2. Pulverförmige Präparate aus einer wasserunlöslichen Kernsubstanz und einer Schutzhülle nach Anspruch 1, enthaltend als wasserunlösliche Kernsubstanz Vitamine oder Carotinoide.

3. Pulverförmige Präparate aus einer wasserunlöslichen Kernsubstanz und einer Schutzhülle nach Anspruch 1 oder 2, erhältlich unter Verwendung von 2 bis 15 Gew.-% der hydrophobierten Kieselsäure,
   bezogen auf die wäßrige Dispersion.

4. Verfahren zur Herstellung von pulverförmigen Präparaten aus einer wasserunlöslichen Kernsubstanz
   und einer Schutzhülle durch Dispergieren der Kernsubstanz in der wäßrigen Lösung eines filmbildenden Polymeren, Versprühen dieser Dispersion unter Mitverwendung eines Sprühhilfsmittels und anschließende Trocknung, dadurch gekennzeichnet, daß man hierbei
   - als filmbildendes Polymeres Amylose oder Stärke mit einem Amylosegehalt über 40 Gew.-% und
   - als Sprühhilfsmittel hydrophobierte Kieselsäure verwendet.

Europäisches
Patentamt

**EUROPÄISCHER RECHERCHENBERICHT**

Nummer der Anmeldung

EP 92 10 9329

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,X | US-A-3 499 962 (O.B. WURZBURG)<br>* Insgesamt *<br>--- | 1-3 | A 61 K 9/16<br>A 61 K 9/50<br>A 61 K 47/36 |
| D,X | GB-A-1 072 795 (EASTMAN KODAK)<br>* Insgesamt *<br>--- | 1-3 | |
| Y | EP-A-0 343 993 (AGRICULTURAL FOOD RESEARCH COUNCIL)<br>* Ansprüche 1-2,7,9-10; Spalte 4, Zeilen 18-29,35-42; Spalte 7, Beispiel 2 *<br>--- | 1-4 | |
| Y | WO-A-9 107 949 (NATIONAL RESEARCH DEVELOPMENT)<br>* Ansprüche 1,3,8; Seite 6, Zeilen 28-35; Seite 7, Zeilen 8-13; Seite 16, Beispiel 4 *<br>--- | 1-4 | |
| D,Y | EP-A-0 074 050 (BASF)<br>* Insgesamt *<br>----- | 1-4 | |

**RECHERCHIERTE SACHGEBIETE (Int. Cl.5)**

A 61 K

Der vorliegende Recherchenbericht wurde für alle Patentansprüche erstellt

| Recherchenort | Abschlußdatum der Recherche | Prüfer |
|---|---|---|
| DEN HAAG | 22-09-1992 | SCARPONI U. |